Europäisches Patentamt

(19) European Patent Office

Office européen des brevets

(11) Publication number: 0 309 086
A1

# (12) EUROPEAN PATENT APPLICATION

(21) Application number: 88307363.7

(22) Date of filing: 09.08.88

(51) Int. Cl.4: A61K 7/06 , A61K 31/20

(30) Priority: 07.09.87 GB 8720984
04.11.87 GB 8725804

(43) Date of publication of application:
29.03.89 Bulletin 89/13

(84) Designated Contracting States:
AT BE CH DE ES FR GB GR IT LI LU NL SE

(71) Applicant: EFAMOL HOLDINGS PLC
Efamol House Woodbridge Meadows
Guildford Surrey GU1 1BA(GB)

(72) Inventor: Horrobin, David Frederick of c/o
Efamol
Holdings PLC Efamol House Woodbridge
Meadows
Guildford Surrey GU1 1BA(GB)
Inventor: Clarkson, Sherri Margaret c/o
Efamol Research Inc.
Annapolis Valley Industrial Park P.O. Box 818
Kentville Nova Scotia, B4N 4H8(CA)

(74) Representative: Caro, William Egerton et al
J. MILLER & CO. Lincoln House 296-302 High
Holborn
London WC1V 7JH(GB)

(54) Treatment of male pattern baldness and of unwanted hair growth.

(57) Method of modulating androgen action in men or women to prevent or treat male pattern baldness and/or reduce or stop unwanted hair growth in areas of the body excluding the scalp, by administering topically or systemically one or more unsaturated fatty acids selected from the 18:3 and higher acids in the n-6 series and 18:4 and higher acids in the n-3 series, in amounts of 1mg to 100g, preferably 50mg to 5g, daily with the limitation that for topical administration the acid(s) are selected from gamma-linolenic acid, dihomo-gamma-linolenic acid and eicosapentaenoic acid.

EP 0 309 086 A1

# TREATMENT OF MALE PATTERN BALDNESS AND OF UNWANTED HAIR GROWTH

## FIELD OF INVENTION

The invention relates to treatment of male pattern baldness and of unwanted hair growth.

## GENERAL DISCUSSION

Essential fatty acid (EFAs) are known to be necessary for the maintenance of normal hair growth. In both animals and humans, dietary EFA deficiency leads to hair loss and the replenishment of EFAs will allow normal growth.

Male pattern baldness is completely different from this type of nutritional hair loss and to our knowledge it has never been shown that such baldness is attributable to a local or general EFA deficiency or that it can be treated by administration of EFAs. In fact even in people with little or no hair on their scalp hair growth on the rest of the body is frequently exuberant, something which could not occur in the presence of an EFA deficiency state. Eunuchs never show male pattern baldness, even in the presence of a strong family history for the trait, while females treated with male hormones may show it. Such baldness is, therefore, generally accepted as due to the action of male hormones (androgens) on the scalp. The most important hormones are probably testosterone and its cutaneous metabolite dihydrotestosterone, but other androgens from the testis, the adrenal and the ovary and produced in the skin may play contributory roles, especially in older women who exhibit hair loss of the male pattern.

Paradoxically, much growth of body hair on areas other than the scalp is in both sexes stimulated rather than inhibited by the actions of androgenic hormones on the hair follicles. Growth of the beard, of pubic and axillary hair, and of hair on the rest of the body is greatly stimulated by the increase in circulating androgens which takes place around the time of puberty. This increase is greater in males than in females but occurs in both sexes. Further increases in androgen levels may occur at other times of life, notably in post-menopausal females. Removal of the circulating androgens by medical or surgical treatment, or antagonism of the action of androgens by drugs such as cyprosterone acetate, are standard techniques for stopping or slowing the growth of androgen-stimulated hair. However, such techniques, which involve substantial manipulation of the hormone environment, are usually restricted to individuals in which the abnormalities are severe. There are no appropriate techniques other than hair removal by shaving, waxing, or other mechanisms which can be used when the degree of hair growth is not sufficient to warrant the use of methods which can cause undesirable side effects.

In men, androgen-stimulated hair growth is not usually a cause of embarrassment, even though hair on the face is regarded as a nuisance which most men remove by shaving. In females, in contrast, with the possible exception of pubic hair, androgen-stimulated hair growth in the axillae, on the legs and arms, on the face, the breasts, the abdomen, the back and on other sites, is commonly regarded as undesirable even when the degree of hair growth is modest. In women in whom such hair growth is obviously excessive, considerable distress can be caused. Facial hair growth in post-menopausal women, associated with the increase in androgens at that time is almost always regarded as undesirable. Some women with unwanted hair growth have higher than normal levels of circulating androgens, but in others the problem seems to be excessive responsiveness of the hair follicles to normal androgen concentrations.

A safe method of reducing such unwanted hair growth on all parts of the body, including the face but excluding the scalp, in both males and females would have considerable value. We propose that such safe reduction of hair growth is possible without administering hormones or using techniques which change the production of the body's own hormones.

The actions of all hormones, including the androgens, depend on the interactions of those hormones with cellular structures known as receptors. There are many types of receptors, each specific for a particular type of hormone. The hair follicles have receptors for androgenic hormones. The combination of a hormone with its specific receptor triggers events which then lead to the characteristic cellular response to the hormone, in this case, hair growth.

It has recently been found that the interactions between several hormones and their specific receptors can be reduced by the presence of unsaturated fatty acids. This has now been found to occur for the female hormones, oestrogen and progesterone, and for the peptide hormones, angiotensin and enkephalin. We have found that control of this interaction can be successfully used in patients to regulate symptoms.

For example, women with premenstrual syndrome have regular cyclic symptoms caused by the changes in hormone levels during the menstrual cycle. Yet the hormone levels found in women with premenstrual syndrome are usually no different from those in women who do not suffer from the syndrome. In the women with the syndrome, there seems to be an abnormal response of the tissues to perfectly normal amounts of circulating hormones. We have found that women with premenstrual syndrome have low plasma levels of polyunsaturated fatty acids. These low levels of the polyunsaturated acids seem to allow excessive amounts of hormones to bind to their receptors: as a result, a normal amount of hormone in the blood produces an excessive response in the tissue. Administration of polyunsaturated fatty acids relieves the symptoms of premenstrual syndrome, apparently by reducing the interaction of the female hormones with their receptors.

This phenomenon has not yet been demonstrated for testosterone or other androgenic hormones. However, in structure there are many similarities between the androgenic hormones and the female hormones and we believe that the effects of androgenic hormones at their receptors are controlled in a similar way, with the greater the degree of the unsaturation of the fatty acid, the greater the effect. The unsaturated fatty acids are thus a safe way of reducing androgen binding to hair follicles. On the scalp this has the effect of stimulating hair growth whereas on the rest of the body including the beard it has the effect of inhibiting hair growth.

The main unsaturated fatty acids in the diet are oleic (1 double bond, 18:1n-9), linoleic (2 double bonds, 18:2n-6) and alpha-linolenic (3 double bonds, 18:3n-3). They are present in adequate amounts in virtually all diets and it is therefore unlikely that oral administration of any one of them would be of much benefit in regulating hair growth, although favourable effects of high oral doses or of topical applications are not ruled out. Linoleic and alpha-linolenic acids are 6-desaturated and then further metabolised in the body to 18:3, 20:3, 20:4, 22:4, 22:5, 24:5 and 24:6 acids of the n-6 series and 18:4, 20:4, 20:5, 22:5, 22:6, 24:6 and 24:7 acids of the n-3 series. It is possible that further elongations and desaturations may occur to a small degree in both series. The structures and conversion paths are:-

| n-6 | | n-3 |
|---|---|---|
| 18:2 delta-9,12 (linoleic acid) | | 18:3 delta-9,12,15 (alpha-linolenic acid) |
| | delta-6 desaturase ↓ | |
| 18:3 delta-6,9,12 (gamma-linolenic acid) | | 18:4 delta-6,9,12,15 |
| | elongation ↓ | |
| 20:3 delta-8,11,14 (didomo-gamma-linolenic acid) | | 20:4 delta-8,11,14,17 |
| | delta-5 desaturase ↓ | |
| 20:4 delta-5,8,11,14 (arachidonic acid) | | 20:5 delta-5,8,11,14,17 |
| | elongation ↓ | |
| 22:4 delta-7,10,13,16 (adrenic acid) | | 22:5 delta-7,10,13,16,19 |
| | delta-4 desaturase ↓ | |
| 22:5 delta-4,7,10,13,16 | | 22:6 delta-4,7,10,13,16,19 |

The acids are in the natural all-cis configuration. In the n-6 series, commonly used names are as follows: for the 18:2 and 18:3 (octadeca di- and tri-enoic) acids linoleic acid and gamma-linolenic acid (GLA); for the 20:3 and 20:4 (eicosatri- and tetraenoic) acids dihomo-gamma-linolenic acid (DGLA) and arachidonic acid (AA); and for the 22:4 (docosatetraenoic) acid adrenic acid. In the n-3 series only alpha-linolenic acid (18:3) is commonly referred to by a non-systematic name but the name stearidonic acid does exist for the 18:4 n-3 acid. Initials are also used e.g. EPA for the 20:5 (eicosapentaenoic) acid.

The initial 6-desaturation step is rate-limiting and can be reduced by many factors, including aging, diabetes, high cholesterol levels, stress which produces high catecholamine levels, zinc deficiency and excess alcohol consumption. It is therefore possible that the supply of unsaturated fatty acids which are 6-desaturated is restricted even in relatively normal adults and improved by the provision of substances which are already 6-desaturated. It should be noted that in modulating hormone action the fatty acids are believed to be in the free form or in other relatively simple form which is able to interact with the receptors. Complex lipids such as phospholipids or triglycerides are unlikely to be active by themselves, but will become active if the fatty acid can be released in the free form, as is possible in most tissues because of enzyme action.

It should be noted however that while all the reactions of EFA metabolism occur in the liver, in other tissues, including the skin, one or more of the enzymes required may be absent. In the skin, for example, the 6- and 5-desaturase enzymes are absent. Thus linoleic acid cannot be converted in the skin to its further metabolites: GLA, if provided directly to the skin can be converted to DGLA, but not further to arachidonic acid. Similarly EPA cannot be made in the skin from its precursors and they must be supplied to the skin in a preformed state.

Although inhibition of steroid hormone binding appears to be a general property of polyunsaturated fatty acids, influenced by the chain length and the number of double bonds, the ability of hair to grow normally will certainly be influenced by other factors. In particular, the circulation in the vicinity of the hair follicles is likely to be important, and many theories of baldness have suggested that restriction in blood flow is an important factor. Among the n-6 and n-3 EFAs, only GLA, DGLA and EPA can, when applied to the skin, be converted to known vasodilator metabolites. GLA can be converted to DGLA and thence to PGE1. EPA can be converted to PGI3. We therefore propose that these three specific fatty acids, alone or in combination, are of particular value in promoting hair growth when applied directly to the skin surface. There is some evidence that the presence of EPA enhances conversion of DGLA to PGE1 in some tissues, while the presence of DGLA enhances conversion of EPA to PGI3. Preparations in which EPA is combined with either GLA or DGLA or both are therefore likely to be particularly desirable.

The salts of the three fatty acids with lithium, sodium or potassium are likely to be particularly desirable in preparations for application to the skin. These salts are soluble in both polar and non-polar solvents and so are able to penetrate both the skin and cells unusually easily. The lithium salts are especially easy to make and to work with, being white crystalline solids, soluble in both aqueous and lipid solvents at room temperature.

## STATEMENT OF INVENTION

As clear from the above we propose broadly that essential fatty acids can be used to modulate androgen action. Specifically we provide:-

(a) Method of modulating androgen action in men or women to prevent or treat male pattern baldness and/or reduce or stop unwanted hair growth in areas of the body excluding the scalp, by administering topically or systemically one or more unsaturated fatty acids selected from the 18:3 and higher acids in the n-6 series and 18:4 and higher acids in the n-3 series, in amounts of 1mg to 100g, preferably 50mg to 5g, daily with the limitation that for topical administration the acid(s) are selected from gamma-linolenic acid, dihomo-gamma-linolenic acid and eicosapentaenoic acid.

(b) Preparation of a medicament for said method, characterised by presenting the said fatty acid or acids, optionally with a diluent or carrier, in unit dosage form suited to topical or systemic (including oral, enteral or parenteral) administration and comprising said amounts of the or each acid.

Preferably n-6 and n-3 series acids are both present.

The acids can be administered as the free acids, triglycerides, other glycerides, phospholipids, ethyl esters, salts, amides, or indeed as any pharmaceutically acceptable derivative which can be shown to raise the levels of the fatty acid in the blood lipids and reference to the acids herein in both description and claims includes such derivatives. For topical administration the free acids and other simple forms such as the metal salts named above are likely to be most effective. Useful derivatives are identified by their having the valuable effect in the body of the acid itself, but conversion can be shown directly by gas chromatographic analysis of concentrations in blood, body fat, or other tissue by standard techniques, for example those of Pelick et al. p. 23, "Analysis of Lipids and Lipoproteins" Ed. Perkins, American Oil Chemists Society, Champaign, Illinois, U.S.A.

## TESTS

In a test of the effectiveness of the invention, four middle-aged males with rapidly progressing baldness were treated orally with either 6g of evening primrose oil or 6g of a mixture of evening primrose oil and concentrated fish oil (20%) per day. The primrose oil provided approximately 90mg of 18:3n-6 (gamma-linolenic acid, GLA) per gram and the fish oil 180mg of 20:5n-3 (eicosapentaenoic acid, EPA) and 120mg of docosahexaenoic acid (DHA) per gram. After delays of 4 to 8 weeks, all four individuals reported unequivocal hair growth, with extension of hair growth to previously bald areas of the scalp, and a strengthening of growth in those areas where hair was present but thinning. Topical application of the unsaturated fatty acids provides better results by increasing the concentration of the fatty acid at the hair follicle level.

In further tests, one male applied pure evening primrose oil twice per day to half the beard area. After about two weeks, the rate of beard growth on the side of the face to which the oil had been applied was definitely less than on the other side. Similarly, one female shaved both legs and then applied evening primrose oil twice daily to one leg only. After a period of 6-8 weeks it could be seen that the rate of hair growth was substantially less on the leg to which the oil had been applied.

These preliminary tests indicate that both oral and topical application of unsaturated fatty acids is able to modulate the actions of androgens on the hair follicles, stimulating hair growth on the scalp and reducing it on other areas of the body.

Oral, intravenous, parenteral, enteral, or suppository or any other systemic presentations may be used and for topical applications oils, emulsions, lotions, solutions, creams, ointments, sticks or any other appropriate formulation all according to methods very well known in themselves.

## EXAMPLES

The following are provided for use against baldness or unwanted bodily hair growth elsewhere than on the scalp:-

1. 1g capsules of conventional soft gelatin containing 800mg of GLA, taken four per day.

2. Similar 1g capsules containing 200mg GLA, 200mg DGLA, 200mg EPA and 200mg DHA, taken three per day.

3. Scalp or body lotion of otherwise conventional cosmetic formulation containing by weight 5% free GLA to be applied once or twice per day in amounts of 1ml per 100 sq. cm. of skin. The lotion should be rubbed in for 2 - 3 minutes and any excess wiped off.

4. Shampoo of otherwise conventional formulation containing by weight 2% free EPA and 2% free GTA to be applied once or twice per day at the rate of 10 - 30 mls per application.

5. Capsules of conventional soft gelatin containing 500mg evening primrose oil and 500mg concentrated fish oil, taken four per day.

6. Cream of otherwise conventional cosmetic formulation containing by weight 5% evening primrose oil and 2% concentrated fish oil to be applied to the scalp or body twice daily once or twice per day in amounts of 1ml per 100 sq. cm. of skin. The lotion should be rubbed in for 2 - 3 minutes and any excess wiped off.

7. Cream of otherwise conventional cosmetic formulation containing by weight 5% lithium-GLA to be applied to the scalp or body twice daily once or twice per day in amounts of 1ml per 100 sq. cm. of skin. The lotion should be rubbed in for 2 - 3 minutes and any excess wiped off.

8. Cream of otherwise conventional cosmetic formulation containing by weight 3% lithium-EPA to be applied to the scalp or body twice daily once or twice per day in amounts of 1ml per 100 sq. cm. of skin. The lotion should be rubbed in for 2 - 3 minutes and any excess wiped off.

9. Cream of otherwise conventional cosmetic formulation containing by weight 3% lithium-GLA and 2% lithium-EPA to be applied to the scalp or body twice daily once or twice per day in amounts of 1ml per 100 sq. cm. of skin. The lotion should be rubbed in for 2 - 3 minutes and any excess wiped off.

## Claims

1. Method of modulating androgen action in men or women to prevent or treat male pattern baldness and/or reduce or stop unwanted hair growth in areas of the body excluding the scalp, by administering topically or systemically one or more unsaturated fatty acids selected from the 18:3 and higher acids in the n-6 series and 18:4 and higher acids in the n-3 series, in amounts of 1mg to 100g, preferably 50mg to 5g, daily with the limitation that for topical administration the acid(s) are selected from gamma-linolenic acid, dihomo-gamma-linolenic acid and eicosapentaenoic acid.

2. Method according to claim 1 in which the fatty acids administered comprise one or more said acids of the n-6 series and one or more said acids of the n-3 series.

3. Method according to claim 1 or 2, the fatty acids being administered topically and being as lithium, sodium or potassium salts.

4. Method of preparation of a medicament for said method as claimed in claim 1, characterised by presenting the said fatty acid or acids, optionally with a diluent or carrier, in unit dosage form suited to topical or systemic (including oral, enteral or parenteral) administration and comprising said amounts of the or each acid.

European. Patent Office

**PARTIAL EUROPEAN SEARCH REPORT**
which under Rule 45 of the European Patent Convention
shall be considered, for the purposes of subsequent
proceedings, as the European search report

Application number

EP 88 30 7363

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 116 439 (SUNTORY) <br><br> * Claims; page 3, line 36 - page 4, line 11; page 7, line 24 - Page 8, line 34; examples 1,4,14,20, 25 * <br> -- | 1-4 | A 61 K 7/06 <br> A 61 K 31/20 |
| X | GB-A-2 151 924 (ROUSSEL-UCLAF) <br><br> * Claims 1,2,4,14,15; page 1, lines 40-45, 62-64 * <br> -- | 1 | |
| X | GB-A-2 150 588 (A. KITANO) <br><br> * Claims; page 1, line 40 - page 2, line 4 * <br> -- | 1-4 | |
| X | FR-A-2 569 347 (M. MOLLET et al.) <br><br> * Claims; page 3,lines 25-29; page 9, line 25 - page 10, line 20; page 10, lines 25-37; page 11, last example * <br> -- <br> ./. | 1-4 | **TECHNICAL FIELDS SEARCHED (Int. Cl.4)** <br><br> A 61 K |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely:

Claims searched incompletely: 1-4

Claims not searched:

Reason for the limitation of the search:

Method for treatment of the human
or animal body by surgery or therapy
(See art. 52(4) of the European
Patent Convention)

| Place of search | Date of completion of the search | Examiner |
|---|---|---|
| The Hague | 14-12-1988 | WILLEKENS |

| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| X | FR-A-2 380 775 (SEDERMA) <br> * Claims * | 1-4 | |
| | ---------- | | |
| | | | TECHNICAL FIELDS SEARCHED (Int. Cl.4) |